(19) Europäisches Patentamt

European Patent Office

Office européen des brevets

(11) **EP 0 988 020 B1**

(12) **EUROPÄISCHE PATENTSCHRIFT**

(45) Veröffentlichungstag und Bekanntmachung des Hinweises auf die Patenterteilung:
**18.02.2004 Patentblatt 2004/08**

(21) Anmeldenummer: **98965728.3**

(22) Anmeldetag: **01.12.1998**

(51) Int Cl.⁷: **A61K 7/06**, A61K 7/48

(86) Internationale Anmeldenummer:
**PCT/EP1998/007794**

(87) Internationale Veröffentlichungsnummer:
**WO 1999/033434 (08.07.1999 Gazette 1999/27)**

(54) **VERWENDUNG VON ANORGANISCH-ORGANISCHEN HYBRIDPREPOLYMEREN**

USE OF INORGANIC-ORGANIC HYBRID PREPOLYMERS

UTILISATION DE PREPOLYMERES HYBRIDES INORGANIQUES-ORGANIQUES

(84) Benannte Vertragsstaaten:
**DE ES FR GB IT**

(30) Priorität: **23.12.1997 DE 19757455**
**20.05.1998 DE 19822722**

(43) Veröffentlichungstag der Anmeldung:
**29.03.2000 Patentblatt 2000/13**

(73) Patentinhaber: **Wella Aktiengesellschaft**
**64274 Darmstadt (DE)**

(72) Erfinder:
• **ALLWOHN, Jürgen-Andreas**
**D-64560 Riedstadt (DE)**
• **BIRKEL, Susanne**
**D-64380 Ro dorf (DE)**
• **BEYER, Angelika**
**D-63857 Waldaschaff (DE)**

(56) Entgegenhaltungen:
**EP-A- 0 159 628**      **EP-A- 0 464 835**
**EP-A- 0 507 469**      **US-A- 4 344 763**

## Beschreibung

Gegenstand der Erfindung ist die Verwendung von mindestens einem anorganisch-organischen Hybridprepolymer in kosmetischen Mitteln sowie die Anwendung von anorganisch-organischen Hybridprepolymeren oder vernetzten anorganisch-organischen Hybridpolymeren zur Behandlung von Haaren, Haut oder Nägeln.

Ein ansprechendes äußeres Erscheinungsbild wurde schon immer als sehr wichtig angesehen. Eine besondere Rolle spielt dabei die Frisur. Basis für ein ansprechendes Äußeres ist gut frisiertes und gepflegtes Haar. Es sind bereits zahlreiche Produkte bekannt, welche den Haaren durch Polymerzusatz Halt, Volumen, Elastizität, Sprungkraft und Glanz verleihen. Diese Stylingprodukte erleichtern z.B. als Gel die Formgebung, verbessern als Haarspray den Stand und als Festigerschaum das Volumen des Haares. Darüber hinaus sollen Stylingprodukte dem Haar neben Halt, natürlicher Sprungkraft und Elastizität einen natürlichen Glanz verleihen.

Mittel zur Festigung der Frisur und Pflege des Haares bestehen üblicherweise aus Lösungen von filmbildenden, synthetischen oder auch natürlichen Polymeren.

Als synthetische Polymere kommen beispielsweise Polyvinylpyrrolidon, Polyvinylpyrrolidon/Polyvinylacetat Copolymer, Polyacrylsäure oder Polymethacrylsäurepolymerisate in Betracht. Von natürlichen Polymeren finden z. B. Schellack, Gelatine, Chitosansalze, Polysaccharide und Derivate, Cellulose und Cellulosederivate Anwendung.

Bezüglich der haarkonditionierenden und insbesondere der haarfestigenden Eigenschaften sind vorstehend genannte Mittel jedoch noch nicht alle völlig zufriedenstellend. Diese Unzulänglichkeiten zeigen sich beispielsweise dadurch, daß es äußerst schwierig ist, alle oder mehrere der gewünschten Anforderungen an Haarbehandlungspolymere gleichzeitig zu erfüllen und daß die herkömmlichen Polymere nach bereits einer Haarwäsche ausgewaschen sind und somit das Volumen und die Frisur komplett verschwunden sind und die Frisur wieder vollständig neu erstellt werden muß.

Es stellt sich deshalb die Aufgabe, den Halt, insbesondere einen langanhaltenden Halt, die Elastizität, die Sprungkraft, den Griff und den Glanz des menschlichen Haares weiter zu verbessern. Diese Aufgabe läßt sich erfindungsgemäß durch die Verwendung von anorganisch-organischen Hybridprepolymeren lösen, welche nach dem Aufbringen auf das Haar zu anorganisch-organischen Hybridpolymeren vernetzt werden.

Es wurde gefunden, daß sich anorganisch-organische Hybridprepolymere auch in kosmetischen Mitteln einsetzen lassen und daß ein Mittel zur länger anhaltenden Festigung der Frisur und Pflege des Haares mit einem Gehalt an mindestens einem anorganisch-organischem Hybridprepolymeren eine deutliche Verbesserung erzielen konnte. Durch die Vernetzung der Prepolymere auf dem Haar sind die Polymere besser mit dem Haar verbunden und können daher auch die Frisur besser stabilisieren. Durch geeignete Wahl der Polymere läßt sich dem Haar zusätzlich zu dem Halt noch guter Griff und Glanz verleihen.

Die durch die Vernetzung der Prepolymere entstehenden anorganisch-organischen Hybridpolymere im Sinne der Erfindung werden auch als ORMOCER®e (Organic Modified Ceramics) bezeichnet. Es handelt sich dabei um Silikonpolymere, welche eine besondere Stellung zwischen den klassisch als anorganisch definierten Gläsern (Silikaten) und organisch vernetzten Polymeren einnehmen. Die Hybridpolymere lassen sich durch den sogenannten Sol-Gel-Prozeß herstellen und sind als Beschichtungsmaterialien für Metalle, Gläser, Steine, Polymere etc. bekannt (Fraunhofer-Institut für Silicatforschung (ISC), Tätigkeitsbericht 1993, Seiten 51 bis 60 und dort zitierte Literatur). Weitere Übersichten über Herstellung, Verwendung und Eigenschaften von anorganisch-organischen Hybridpolymeren sind beschrieben in "Síntesis y preparación de materiales hibridos orgánico/inorgánico.Ormoceros.Nuevas aplicaciones de los materiales polimeros"; Revista de Plásticos Modernos, Nummer 483, September 1996, Seiten 257 bis 274 und dort zitierte Literatur sowie in "ORMOCER: Neuer Korrosionsschutz für Messingoberflächen", Jahrbuch Oberflächentechnik (1993), 49, Seiten 243 bis 251.

Verfahren zur Herstellung von anorganisch-organischen Hybridpolymeren und -prepolymeren sowie deren Anwendung als Beschichtungsmaterial für u.a. Metalloberflächen oder Geweben aus Naturfasern sind beispielsweise beschrieben in der DE-OS 38 28 098, EP 0 526 875, EP 0 580 488, EP 0 610 831, EP 0 792 846 und in der WO 95/13855.

Zur Synthese der Hybridprepolymere werden funktionalisierte Silane der allgemeinen Formel (I)

$$RSiX_3 \tag{I}$$

eingesetzt, wobei X für eine hydrolysierbare und kondensierbare Gruppe und R für einen vernetzbaren organischen Rest steht. Durch hydrolytische Vorkondensation wird zunächst das anorganische Si-O-Si-Netzwerk gebildet, welches anschließend über Reaktionen der vernetzbaren organischen R-Gruppen weiter vernetzt wird.

Unter Hybridprepolymeren im Sinne der vorliegenden Erfindung werden die durch Vorkondensation erhaltenen Produkte verstanden, welche noch nicht über die R-Gruppen vernetzt sind.

Der vernetzbare Rest R bedeutet eine meist aliphatische Seitengruppe, die unterschiedliche Funktionalitäten wie Amino-, Epoxy-, Hydroxy-, Methacrylat- oder andere polymerisierbare Gruppen enthalten kann. R kann insbesondere ausgewählt sein aus Alkyl, Alkenyl, Alkinyl, Aryl, Arylalkyl, Alkylaryl, Arylalkenyl, Alkenylaryl, Arylalkinyl, Alkinylaryl, wobei der Rest durch O-, S-oder N-Atome unterbrochen sein kann und, wenn er nicht selbst schon vernetzbar ist, mindestens einen vernetzbaren Substituenten aus der Gruppe der Halogene, Amino-, Amid-, Aldehyd-, Keto-, Alkylcarbonyl-, Carboxy-, Mercapto-, Cyano-, Hydroxy-, Alkoxy-, Methacryloxy, Epoxy- oder Vinylgruppen enthält.

Die Gruppen X können unabhängig voneinander Alkoxy-, Aryloxy-, Acyloxy-, Alkylcarbonyl-, Alkoxycarbonylgruppen, Halogen, Wasserstoff oder substituierte oder unsubstituierte Aminogruppen bedeuten, wobei Ethoxyund Methoxygruppen besonders bevorzugt sind.

Das Silan der Formel (I) kann insbesondere ausgewählt sein aus aus Vinyltrialkoxysilan, Vinyltriacetoxysilan, Aminopropyltrialkoxysilan, Isocyanatopropyltrialkoxysilan, Mercaptopropyltrialkoxysilan, Vinyltrichlorsilan, Allyltrialkoxysilan, Allyltriacetoxysilan, 3-Isocyanatooxypropyltrialkoxysilan, Methacryloxypropenyltrialkoxysilan, 3-Methacrylcarbonyloxypropyltrialkoxysilan, p-Aminophenyltrialkoxysilan, 3-Aminopropyltrialkoxysilan, 3-Cyanopropyltrialkoxysilan, 4-Mercaptobutyltrialkoxysilan, 6-Mercaptohexyltrialkoxysilan, 3-Mercaptopropyltrialkoxysilan, 3-(Ethylendiamino)propyltrialkoxysilan, 3-(Diethylentriamino)propyltrialkoxysilan, 3-Glycidoxypropyltrialkoxysilan, 2-[4-(1,2-Epoxycyclohexyl)]ethyltrialkoxysilan und 3-(Trialkoxysilyl)propylbernsteinsäureanhydrid, wobei Alkoxygruppen Methoxy- oder Ethoxygruppen bedeuten. Besonders bevorzugt sind 3-Glycidoxypropyltrialkoxysilan und 3-Mercaptopropyltrialkoxysilan.

Anstelle der monomeren Ausgangssilane können auch vorkondensierte, im Reaktionsmedium lösliche Oligomere der Silane eingesetzt werden. Desweiteren können auch fluorierte Derivate der Silane eingesetzt werden.

Die Verbindungen der allgemeinen Formel (I) können mit Metallverbindungen wie beispielsweise den Alkyl-, Alkoxy-, Halogen-, Acyloxy-, Hydroxy-, Oxyhalogen- oder Hydroxyhalogenverbindungen von Titan, Zirkonium oder Aluminium, sowie mit weiteren, netzwerkmodifizierenden Verbindungen, beispielsweise der Formel $SiX_4$ (II) oder Verbindungen der Formel $SiR'X_2R$ (III) kombiniert werden, wobei R' für eine nicht vernetzbare und nicht kondensierbare Alkyl- oder Arylgruppe steht und R und X die oben angegebenen Bedeutungen haben.

Die netzwerkmodifizierenden Verbindungen können beispielsweise im Falle von Verbindungen der Formel (II) eine Erhöhung des Vernetzungsgrades im anorganischen Teil oder im Falle von Verbindungen des Typs (III) dessen Verringerung bewirken.

Die Metallverbindungen der Übergangsmetalle, insbesondere der IV. Nebengruppe, bevorzugt Ti- oder Zr-Verbindungen oder der III. oder IV. Hauptgruppe, bevorzugt Aluminiumverbindungen, können insbesondere ausgewählt sein aus Tetrachlortitan, Tetraalkoxytitan, Tetrachlorzirkonium, Tetraalkoxyzirkonium, Dichlorzirkoniumoxid, Trialkoxyaluminium und Dihydroxyaluminiumchlorid Tributoxyaluminium und Tetrapropoxyzirkonium, wobei Alkoxy für Methoxy, Ethoxy, i- oder n-Propoxy, Butoxy oder 2-Ethylhexoxy steht.

Die Verbindung der Formel (II) kann insbesondere ausgewählt sein aus Tetramethoxysilan, Tetraethoxysilan, Trimethoxysilan, Tetra-n- oder Tetra-ipropoxysilan, Tetrabutoxysilan, Tetrachlorsilan, Trichlorsilan, Tetraacetoxysilan.

Weitere netzwerkmodifizierende Verbindungen können außerdem ausgewählt sein aus Methyltrichlorsilan, Methyltrialkoxysilan, Ethyltrichlorsilan, Ethyltrialkoxysilan, Propyltrialkoxysilan, Phenyltrialkoxysilan, Dimethyldichlorsilan, Dimethyldialkoxysilan, Dimethyldihydroxysilan, Diphenyldichlorsilan, Diphenyldialkoxysilan, Tripropylhydroxysilan, 4-Aminobutylmethyldialkoxysilan, Aminomethyldimethylalkoxysilan, Vinylethyldichlorsilan, Vinylmethyldiacetoxysilan, Vinylmethyldichlorsilan, Vinylmethyldialkoxysilan, Phenylvinydialkoxysilan, Phenylallyldichlorsilan, 4-Aminobutylmethyl-dialkoxysilan und Aminomethyldimethylalkoxysilan und vergleichbaren Verbindungen, wobei Alkoxygruppen vorzugsweise Methoxy- oder Ethoxygruppen bedeuten.

Herstellung und Anwendung der anorganisch-organischen Hybridpolymere erfolgt durch die Hydrolyse der Ausgangsverbindungen zu einer kolloidalen Lösung, die die abgespaltenen Hydrolyseprodukte, beispielsweise die Alkohole enthält und auch als Lack bezeichnet wird. Dieser Lack kann entweder direkt auf das Substrat (z.B. Haar, Haut oder Nägel) aufgebracht oder er kann in übliche kosmetische Mittel eingearbeitet werden. In einem zweiten Schritt erfolgt nach der Bildung des anorganischen Si-O-Si-Netzwerks die Vernetzung der organischen Molekülgruppen miteinander. Dies kann durch herkömmliche Polymerisationsreaktionen, beispielsweise durch Reaktionen von Doppelbindungen erfolgen. Auch Polyadditionen, wie sie von Epoxidharzen bekannt sind, können zu einer Vernetzung der organischen Seitenketten genutzt werden.

Vorzugsweise erfolgt die Vorkondensation in Gegenwart eines Kondensationskatalysators. Als Kondensationskatalysatoren eignen sich Protonen oder Hydroxylionen abspaltende Verbindungen und Amine. Hinsichtlich der Verfahrensführung mittels Vorkondensation wird auf die DE-OS 38 28 098 Bezug genommen. Die vorliegende Erfindung schließt hierbei auch die in der vorstehenden Druckschrift genannten Kondensationskatalysatoren ein.

Die Anwendung von anorganisch-organischen Hybridprepolymeren zur Haarbehandlung kann in der Weise erfolgen, daß in einem ersten Schritt eine hydrolytische Vorkondensation, gegebenenfalls in Anwesenheit mindestens eines Kondensationskatalysators, von mindestens einem organofunktionellem Silan der Formel (I) erfolgt und in einem zweiten Schritt die Vernetzung zum Hybridpolymeren stattfindet. Die Vernetzung kann dabei durch Wärme, durch Licht oder durch geeignete, übliche Polymerisationsinitiatoren induziert werden.

Die Vernetzung erfolgt vorzugsweise nach dem Aufbringen auf das Haar.

Die Hybridprepolymere bieten in der Form des anorganischen Netzwerkes (Vorkondensat des ersten Reaktionsschrittes) die Möglichkeit, sich in Mittel zur Festigung und Pflege der Haare auf wäßriger, alkoholischer oder wäßrig/alkoholischer Basis einarbeiten und auf das Haar aufbringen zu lassen. Durch Wärmeeinwirkung wird dann das organische Netzwerk auf dem Haar gebildet und fixiert so die Frisur. Je nach dem, wie die Eigenschaften dieses Polysiloxanes eingestellt werden, wird ein festerer oder gepflegterer Halt des Haares und ein angenehmer, natürlicher Griff mit viel Glanz auf dem Haar erhalten.

Bevorzugte Systeme zur Bildung der anorganisch-organischen Hybridpolymere sind:

1.) 3-Glycidoxypropyl-trimethoxysilan, 3-Triethoxysilylpropyl-bernsteinsäureanhydrid, und 1-Methylimidazol; vorzugsweise im Massenverhältnis von 45 bis 65 : 30 bis 45 : 1; z.B. 57,4 : 36,9 : 1

2.) 3-Glycidoxypropyl-trimethoxysilan, Trimethoxyphenylsilan, Aminosilan z.B. Aminopropyltriethoxysilan, Tributoxyaluminium, Acetessigsäureethylester; vorzugsweise im Massenverhältnis von 1 bis 2 : 1,5 bis 2,5 : 1 : 4 bis 5 : 2 bis 3; z.B. 1,4 : 1,8 : 1 : 4,45 : 2,75

3.) 3-Glycidoxypropyl-trimethoxysilan, Tetramethoxysilan, Tributoxyaluminium, Tetrapropoxyzirkonium, Triethanolamin; vorzugsweise im Massenverhältnis von 4,5 bis 5,5 : 2 bis 3 : 0,8 bis 1,8 : 1,7 bis 2,5 : 1; z.B. 5,1 : 2,5 : 1,2 : 2,1 : 1

4.) 3-Glycidoxypropyl-trimethoxysilan, Trimethoxyphenylsilan, 2,2,2-Trifluoroethylamin, Tributoxyaluminium, Acetessigsäureethylester; vorzugsweise im Massenverhältnis von 13 bis 16 : 1,5 bis 2,5 : 1 : 3 bis 5 : 2 bis 3,5; z.B. 14,4 : 1,9 : 1 : 4,0 : 2,6

5.) 3-Methacryloxypropyltrimethoxysilan, Tetrapropoxyzirkonium, vorzugsweise im Massenverhältnis von 2 bis 3,5 : 1; z.B. 2,6 : 1 sowie mit UV-Initiator oder thermischem Initiator;

6.) 3-Glycidoxypropyl-trimethoxysilan, Trimethoxyphenylsilan, Aminosilan z.B. Aminopropyltriethoxysilan, Tributoxyaluminium, Acetessigsäureethylester; vorzugsweise im Massenverhältnis von 6 bis 9 : 1 : 0,8 bis 1,5 : 2 bis 3 : 1 bis 2; z.B. 7,2 : 1 : 1,1 : 2,5 : 1,3

7.) 3-Glycidoxypropyl-trimethoxysilan, Trimethoxyphenylsilan, Aminosilan z.B. Aminopropyltriethoxysilan, Tributoxyaluminium, Tetrapropoxyzirkonium; vorzugsweise im Massenverhältnis von 9 bis 10,4 : 3,8 bis 4,5 : 1 : 0,8 bis 2,8 : 3 bis 5; z.B. 9,6 : 4,1 : 1 : 2,2 : 3,9

8.) 3-Glycidoxypropyl-trimethoxysilan, Aminosilan z.B. Aminopropyltriethoxysilan, 3-Mercaptopropyltriethoxysilan; vorzugsweise im Massenverhältnis von 18 bis 21 : 1 : 6,5 bis 8; z.B. 19,2 : 1 : 7,2

9.) Mercaptopropyltriethoxysilan, Salzsäure (1N) im molaren Verhältnis von vorzugsweise 1 : 1 bis 2; z.B. 1 : 1,5

10.) 3-Glycidoxypropyl-trimethoxysilan, Trimethoxyphenylsilan, Tributoxyaluminium, Acetessigsäureethylester; vorzugsweise im Massenverhältnis von 5 bis 6 : 1 bis 2 : 1 bis 2,5 : 1; z.B. 5,4 : 1,5 : 1,9 : 1

11.) Mercaptopropyltriethoxysilan und Vinyltriethoxysilan vorzugsweise im Massenverhältnis von 1 bis 2 : 1; z.B. 1,25 : 1 sowie wässrige Salzsäure (1N).

Besonders bevorzugt sind die Systeme 3.), 9.) und 11.).

Vorteilhafterweise kann das anorganisch-organische Hybridpolymer oder -prepolymer auch als Träger für kosmetische oder pharmazeutische Wirk- und Hilfsstoffe wie z.B. Anti-Schuppenmittel, Farbstoffe oder auch Pigmente eingesetzt werden. Die Wirk- oder Hilfsstoffe können hierbei entweder physikalisch eingeschlossen oder chemisch gebunden sein.

Gegenstand der Erfindung ist auch ein Verfahren zur Haarbehandlung, bei welchem

a) ein noch nicht vernetztes Vorkondensationsprodukt eines anorganisch-organischen Hybridpolymers in einer geeigneten kosmetischen Grundlage auf das Haar aufgebracht wird und
b) anschließend zum Hybridpolymer vernetzt wird.

Die Vernetzung kann hierbei durch Wärme, Licht oder Polymerisationsinitiatoren induziert werden. Geeignete Initiatoren sind beispielsweise 1-Hydroxy-cyclohexylphenylketon oder tert.-Butylperoxy-2-ethylhexanoat. Eine thermisch induzierte Vernetzung erfolgt vorzugsweise bei 20 bis 80°C, besonders bevorzugt bei 40 bis 50°C innerhalb von vorzugsweise 2 bis 20 Minuten.

Gegenstand der vorliegenden Erfindung ist auch ein kosmetisches Mittel mit einem Gehalt an mindestens einem anorganisch-organischen Hybridprepolymer in einer geeigneten kosmetischen Grundlage. In einem erfindungsgemäßen kosmetischen Mittel ist das Hybridprepolymer in einer Menge von vorzugsweise 0,01 bis 40 Gewichtsprozent, besonders bevorzugt in einer Menge von 0,05 bis 15 Gewichtsprozent in einer geeigneten kosmetischen Grundlage enthalten.

Das erfindungsgemäße Mittel liegt im allgemeinen als wäßrige, alkoholische oder wäßrig-alkoholische Lösung vor. Geeignete Lösungsmittel sind beispielsweise aliphatische Alkohole mit 1 bis 4 Kohlenstoffatomen oder ein Gemisch von Wasser mit einem der genannten Alkohole. Es können jedoch auch andere organische Lösungsmittel eingesetzt werden, wobei insbesondere unverzweigte oder verzweigte Kohlenwasserstoffe wie Pentan, Hexan, Isopentan und zyklische Kohlenwasserstoffe wie Cyclopentan und Cyclohexan zu nennen sind. Die Lösungsmittel liegen in einer Menge von 0,5 bis 99 Gewichtsprozent, bevorzugt in einer Menge von 40 bis 90 Gewichtsprozent vor.

In einer besonderen Ausführungsform wird das anorganisch-organische Hybridprepolymer zusammen mit mindestens einem filmbildenden und haarfestigenden Polymeren eingesetzt. Das filmbildende und haarfestigende Polymer kann synthetischen oder natürlichen Ursprungs sein und nichtionischen, kationischen, anionischen oder amphoteren Charakter haben. Ein derartiger Polymerzusatz, der in Mengen von 0,01 bis 50 Gewichtsprozent, vorzugsweise in Mengen von 0,01 bis 20 Gewichtsprozent, besonders bevorzugt von 0,1 bis 15 Gewichtsprozent im Haarbehandlungsmittel enthalten ist, kann auch aus einem Gemisch von mehreren Polymerern bestehen und durch den Zusatz von weiteren Polymeren mit verdickender Wirkung in seinen haarfestigenden Eigenschaften noch modifiziert werden.

Unter filmbildenden, haarfestigenden Polymeren werden erfindungsgemäß solche Polymere verstanden, die bei Anwendung in 0,01 bis 5%-iger wäßriger, alkoholischer oder wäßrig-alkoholischer Lösung in der Lage sind, auf dem Haar einen Polymerfilm abzuscheiden und auf diese Weise das Haar zu festigen.

Als geeignete synthetische, nichtionische, filmbildende, haarfestigende Polymere können in dem erfindungsgemäßen Haarbehandlungsmittel Homopolymere des Vinylpyrrolidons, Homopolymere des N-Vinylformamids, Copolymerisate aus Vinylpyrrolidon und Vinylacetat, Terpolymere aus Vinylpyrrolidon, Vinylacetat und Vinylpropionat, Polyacrylamide, Polyvinylalkohole, oder Polyethylenglykole mit einem Molekulargewicht von 800 bis 20.000 g/mol eingesetzt werden.

Unter den geeigneten synthetischen, filmbildenden anionischen Polymeren sind zu nennen Crotonsäure/Vinylacetat Copolymere und Terpolymere aus Acrylsäure, Ethylacrylat und N-t-Butylacrylamid.

Natürliche filmbildende Polymere oder daraus durch chemische Umwandlung hergestellte Polymere können in dem erfindungsgemäßen Haarbehandlungsmittel ebenfalls eingesetzt werden. Bewährt haben sich niedermolekulares Chitosan mit einem Molekulargewicht von 30.000 bis 70.000 g/mol oder hochmolekulares Chitosan, Gemische aus Oligo-, Mono- und Disacchariden, chinesisches Balsamharz, Cellulosederivate wie Hydroxypropylcellulose mit einem Molekulargewicht von 30.000 bis 50.000 g/mol, oder Schellack in neutralisierter oder unneutralisierter Form.

Auch amphotere Polymere können in dem erfindungsgemäßen Haarbehandlungsmittel eingesetzt werden. Geeignet sind zum Beispiel Copolymere aus Octylacrylamid, t-Butylaminoethylmethacrylat sowie zwei oder mehr Monomeren aus der Gruppe Acrylsäure, Methacrylsäure und deren einfachen Estern.

Unter den kationischen Polymeren, die erfindungsgemäß eingesetzt werden können, sind Copolymere des Vinylpyrrolidons mit quaternierten Derivaten des Dialkylaminoacrylats- und -methacrylats, wie beispielsweise mit Diethylsulfat quaternierte Vinylpyrrolidon/ Dimethylaminomethacrylat Copolymere zu nennen. Weitere kationische Polymere sind beispielsweise das Copolymerisat des Vinylpyrrolidons mit Vinylimidazoliummethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcarprolactam, das quaternierte Ammoniumsalz, hergestellt aus Hydroxyethylcellulose und einem mit Trimethylammonium substituierten Epoxid, das Vinylpyrrolidon/Methacrylamidopropyltrimethylammoniumchlorid Copolymer und diquaternäre Polydimethylsiloxane.

Die Konsistenz des erfindungsgemäßen Haarbehandlungsmittels kann durch den Zusatz von Verdickern erhöht werden. Hierfür sind beispielsweise Homopolymere der Acrylsäure mit einem Molekulargewicht von 2.000.000 bis 6.000.000 g/mol geeignet. Auch Copolymere aus Acrylsäure und Acrylamid (Natriumsalz) mit einem Molekulargewicht von 2.000.000 bis 6.000.000 g/mol und Sclerotium Gum sind geeignet. Auch geeignet sind Copolymere der Acrylsäure und der Methacrylsäure.

Üblicherweise können dem erfindungsgemäßen Haarbehandlungsmittel weitere bekannte kosmetische Zusatzstoffe beigefügt werden, zum Beispiel nichtfestigende, nichtionische Polymere wie Polyethylenglykol mit einem Molekulargewicht von etwa 600 g/mol, nichtfestigende, anionische und natürliche Polymere sowie deren Mischungen in einer Menge von vorzugsweise 0,01 bis 50 Gewichtsprozent. Auch Parfümöle in einer Menge von 0,01 bis 5 Gewichtsprozent, Trübungsmittel wie Ethylenglykoldistearat in einer Menge von 0,01 bis 5 Gewichtsprozent, Netzmittel oder Emulgatoren aus den Klassen der anionischen, kationischen, amphoteren oder nichtionogenen Tenside wie Fettalkoholsulfate, ethoxylierte Fettalkohole, Fettsäurealkanolamide wie die Ester der hydrierten Rizinusölfettsäuren in einer Menge von 0,1 bis 30 Gewichtsprozent, außerdem Feuchthaltemittel, Farbstoffe, Lichtschutzmittel, Antioxidantien und Konservierungsstoffe in einer Menge von 0,01 bis 10 Gewichtsprozent.

Das erfindungsgemäße Haarbehandlungsmittel kann weiterhin durch Zusatz von herkömmlichen Silikonpolymeren verbessert werden, wie beispielsweise Polydimethylsiloxan (INCI: Dimethicon), α-Hydro-ω-hydroxypolyoxydimethylsilylen (INCI: Dimethiconol), cyclisches Dimethylpolysiloxan (INCI: Cyclomethicon), Trimethyl(octadecyloxy)silan (INCI: Stearoxytrimethylsilan), Dimethylsiloxan/Glykol Copolymer (INCI: Dimethicon Copolyol), Dimethylsiloxan/Aminoalkylsiloxan Copolymer mit Hydroxyendgruppen (INCI: Amodimethicon), Monomethylpolysiloxan mit Laurylseitenketten und Polyoxyethylen- und/oder Polyoxypropylenendketten, (INCI: Laurylmethicon Copolyol), Dimethylsiloxan/Glykol Copolymeracetat (INCI: Dimethiconcopolyol Acetat), Dimethylsiloxan/Aminoalkylsiloxan Copolymer mit Trimethylsilylendgruppen (INCI: Trimethylsilylamodimethicon). Bevorzugte Silikonpolymere sind Dimethicone, Cyclomethicone und Dimethiconole. Auch Mischungen von Silikonpolymeren sind geeignet wie zum Beispiel eine Mischung aus Dimethicon und Dimethiconol.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Das erfindungsgemäße Mittel kann in verschiedenen Applikationsformen Anwendung finden, wie beispielsweise in Aerosolzubereitungen als Schaum oder als Spray, desweiteren als Non-Aerosol, welches mittels einer Pumpe oder als "Pump and Spray" zum Einsatz kommt. Der Einsatz in üblichen O/W und W/O Emulsionen ist ebenso möglich wie in Anwendungsformen als Lotion, Milch, Flüssigfestiger, Creme, Gel, Gelschaum, Wachs oder Mikroemulsion.

Das erfindungsgemäße Mittel kann auch als färbendes oder pflegendes Haarbehandlungsmittel wie zum Beispiel als Farbfestiger und Haarspülung formuliert sein.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt. Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Unter Haarbehandlung soll die Behandlung des menschlichen Kopfhaares vor allem zum Zweck der Herstellung einer Frisur oder zur Pflege der Haare verstanden werden.

Die nachfolgenden Beispiele sollen den Gegenstand der Erfindung näher erläutern. Die beispielhaft beschriebenen haarfestigenden Mittel zeichnen sich insbesondere durch eine langanhaltende Haarfestigung aus. Nach dem ersten Auswaschen bleibt das Polymernetzwerk im Haar haften und führt zu einer immer noch anhaltenden Festigung. Je nach gewähltem System kann drei bis fünf mal Waschen genügen um den unbehandelten Zustand des Haares wiederherzustellen.

| **Beispiel 1 :** Haarfestigungsmittel | |
|---|---|
| 1,50 g | anorganisch-organisches Hybridprepolysiloxan aus 3-Glycidoxypropyl-trimethoxysilan, 3-Triethoxysilylpropylbern-steinsäureanhydrid und 1-Methylimidazol (System 1.)) |
| 1,50 g | Vinylpyrrolidon/Vinylacetat Copolymer |
| 0,20 g | 1,2-Propylenglykol |
| 0,15 g | Parfüm |
| 0,03 g | Cetyltrimethylammoniumchlorid |
| 20,21 g | Wasser |
| 76,41 g | Ethanol |
| 100,00 g | |

Alternativ kann als Hybridprepolymer auch jedes andere der der oben beschriebenen Systeme 2.) bis 10.) eingesetzt werden.

| **Beispiel 2:** Haarfestigungsmittel | |
|---|---|
| 0,88 g | anorganisch-organisches Hybridprepolysiloxan aus Mercaptopropyltriethoxysilan und Salzsäure (System 9.)) |
| 2,63 g | Vinylpyrrolidon/Vinylacetet Copolymer |
| 0,20 g | 1,2-Propylenglykol |
| 0,15 g | Parfüm |

(fortgesetzt)

| Beispiel 2: Haarfestigungsmittel | |
| --- | --- |
| 0,05 g | Cetyltrimethylammoniumchlorid |
| 59,89 g | Wasser |
| 46,28 g | Ethanol |
| 100,00 g | |

| Beispiel 3: Haarfestigungsmittel | |
| --- | --- |
| 4,88 g | anorganisch-organisches Hybridprepolysiloxan aus 3-Glycidoxypropyl-trimethoxysilan, Tetramethoxysilan, Tributoxyaluminium, Tetrapropoxyzirkonium und Triethanolamin (System 3.)) |
| 0,20 g | 1,2-Propylenglykol |
| 0,15 g | Parfüm |
| 0,05 g | Cetyltrimethylammoniumchlorid |
| 49,71 g | Wasser |
| 45,01 g | Ethanol |
| 100,00 g | |

| Beispiel 4: Fönlotion mit UV-Schutz | |
| --- | --- |
| 1,80 g | anorganisch-organisches Hybridprepolysiloxan aus 3-Glycidoxypropyltrimethoxysilan, Trimethoxyphenylsilan, 2,2,2-Trifluoroethylamin, Tributoxyaluminium und Acetessigsäureethylester (System 4.)) |
| 1,20 g | Polyvinylpyrrolidon |
| 0,20 g | Parfüm |
| 0,15 g | Glycerin (85prozentig) |
| 0,10 g | 2-Hydroxy-4-methoxybenzophenon |
| 61,30 g | Wasser |
| 35,25 g | Ethanol |
| 100,00 g | |

| Beispiel 5: Haarpflegende Festigerlotion | |
| --- | --- |
| 2,12 g | anorganisch-organisches Hybridprepolysiloxan aus Mercaptopropyltriethoxysilan und Salzsäure (System 9.)) |
| 2,12 g | vinylpyrrolidon/Vinylacetat Copolymer |
| 0,40 g | Hydriertes Rizinusöl, ethoxyliert mit 40 Mol Ethylenoxid |
| 0,20 g | Parfüm |
| 95,16 g | Wasser |
| 100,00 g | |

| Beispiel 6: Schaumfestiger mit starker Festigung | |
| --- | --- |
| 2,00 g | anorganisch-organisches Hybridprepolysiloxan aus Mercaptopropyltriethoxysilan und Salzsäure (System 9.)) |
| 2,00 g | Vinylpyrrolidon/Methylaminoethylmethacrylat Copolymer |
| 0,45 g | Glyceryllaurat |
| 0,15 g | Parfüm |
| 0,16 g | Cetyltrimethylammoniumchlorid |

(fortgesetzt)

| Beispiel 6: Schaumfestiger mit starker Festigung | |
|---|---|
| 5,00 g | Propan/Butan (5,0 bar) |
| 14,95 g | Ethanol |
| 75,29 g | Wasser |
| 100,00 g | |

| Beispiel 7: Schaumfestiger | |
|---|---|
| 3,00 g | anorganisch-organisches Hybridprepolysiloxan aus 3-Glycidoxypropyltrimethoxysilan, Tetramethoxysilan, Tributoxyaluminium, Tetrapropoxyzirkonium und Triethanolamin (System 3.)) |
| 1,00 g | Vinylpyrrolidon/Methylaminoethylmethacrylat Copolymer |
| 0,20 g | 1,2-Propylenglykol |
| 0,17 g | Parfüm |
| 0,10 g | Cetyltrimethylammoniumchlorid |
| 6,00 g | Propan/Butan (5,0 bar) |
| 18,66 g | Ethanol |
| 70,87 g | Wasser |
| 100,00 g | |

| Beispiel 8: Schaumfestiger | |
|---|---|
| 4,00 g | anorganisch-organisches Hybridprepolysiloxan aus Mercaptopropyltriethoxysilan und Salzsäure (System 9.)) |
| 1,00 g | anorganisch-organisches Hybridprepolysiloxan aus 3-Glycidoxypropyl-trimethoxysilan, Tetramethoxysilan, Tributoxyaluminium, Tetrapropoxyzirkonium und Triethanolamin (System 3.)) |
| 0,20 g | 1,2-Propylenglykol |
| 0,17 g | Parfüm |
| 0,10 g | Cetyltrimethylammoniumchlorid |
| 6,00 g | Propan/Butan (5,0 bar) |
| 69,04 g | Ethanol |
| 19,49 g | Wasser |
| 100,00 g | |

| Beispiel 9: Pflegender Schaumfestiger | |
|---|---|
| 1,12 g | anorganisch-organisches Hybridprepolysiloxan aus 3-Glycidoxypropyltrimethoxysilan, Amino-silan und 3-Mercaptopropyltriethoxysilan (System 8.)) |
| 3,40 g | Vinylcaprolactam/Vinylpyrrolidon/Dimethylaminoethyl-methacrylat Terpolymer |
| 0,60 g | Ameisensäure |
| 0,60 g | Hydriertes Rizinusöl, ethoxyliert mit 40 Mol Ethylenoxid |
| 0,22 g | Decylpolyglucosid |
| 0,09 g | Cetyltrimethylammoniumchlorid |
| 0,20 g | Parfüm |
| 6,00 g | Propan/Butan (5,0 bar) |
| 87,77 g | Wasser |
| 100,00 g | |

| **Beispiel 10:** Styling-Haarspray | |
|---|---|
| 3,14 g | anorganisch-organisches Hybridprepolysiloxan aus Mercaptopropyltriethoxysilan und Salzsäure (System 9.)) |
| 1,50 g | Octylacrylamid/Acrylsäure/Butylaminoethylmethacrylat/ Methacrylat/Hydroxypropylmethacrylat Copolymer |
| 0,15 g | Parfüm |
| 10,67 g | Butan (1,5 bar) |
| 33,33 g | Propan/Butan |
| 51,21 g | Ethanol |
| 100,00 g | |

| **Beispiel 11:** Pumpspray | |
|---|---|
| 2,79 g | anorganisch-organisches Hybridprepolysiloxan aus Mercaptopropyltriethoxysilan, Vinyltriethoxysilan und wässrige Salzsäure (1N) (System 11.)) |
| 0,30 g | Parfüm |
| 0,10 g | Dimethylsiloxan/Ethylenglykol Copolymer |
| 11,53 g | Wasser |
| 85,28 g | Ethanol |
| 100,00 g | |

| **Beispiel 12:** 80% VOC-Haarspray | |
|---|---|
| 1,63 g | anorganisch-organisches Hybridprepolysiloxan aus 3-Glycidoxypropyl-trimethoxysilan, Tetramethoxysilan, Tributoxyaluminium, Tetrapropoxyzirkonium und Triethanolamin (System 3.)) |
| 0,82 g | anorganisch-organisches Hybridprepolysiloxan aus Mercaptopropyltriethoxysilan und Salzsäure (System 9.)) |
| 4,00 g | Vinylacetat/Crotonsäure/Polyethylenoxid Copolymer |
| 0,20 g | Cyclo-Tetra(dimethylsiloxan) |
| 0,15 g | Parfüm |
| 13,20 g | Wasser |
| 40,00 g | Ethanol |
| 40,00 g | Dimethylether |
| 100,00 g | |

| **Beispiel 13:** 80% VOC-Pumpspray | |
|---|---|
| 1,50 g | anorganisch-organisches Hybridprepolysiloxan aus Mercaptopropyltriethoxysilan, Vinyltriethoxysilan und wässrige Salzsäure (1N) (System 11.)) |
| 1,50 g | anorganisch-organisches Hybridprepolysiloxan aus 3-Glycidoxypropyl-trimethoxysilan, Tetramethoxysilan, Tributoxyaluminium, Tetrapropoxyzirkonium und Triethanolamin (System 3.)) |
| 5,00 g | Vinylacetat/Crotonsäure/Polyethylenoxid Copolymer |
| 0,30 g | Parfüm |
| 11,70 g | Wasser |
| 80,00 g | Ethanol |
| 100,00 g | |

| | **Beispiel 14:** 55% VOC-Pumpspray |
|---|---|
| 4,00 g | anorganisch-organisches Hybridprepolysiloxan aus 3-Glycidoxypropyl-trimethoxysilan, Tetramethoxysilan, Tributoxyaluminium, Tetrapropoxyzirkonium und Triethanolamin (System 3.)) |
| 0,20 g | Parfüm |
| 40,80 g | Wasser |
| 55,00 g | Ethanol |
| 100,00 g | |

| | **Beispiel 15:** Haargel |
|---|---|
| 2,76 g | anorganisch-organisches Hybridprepolysiloxan aus 3-Glycidoxypropyl-trimethoxysilan, Trimethoxyphenylsilan, Aminosilan, Tributoxyaluminium und Tetrapropoxyzirkonium (System 7.)) |
| 0,40 g | Polyacrylsäure |
| 0,10 g | Hydroxypropylmethylcellulose |
| 0,80 g | Polyoxyethylen-(20)-sorbitanmonopalmitat |
| 0,50 g | Polyoxyethylen-(25)-p-aminobenzoesäure |
| 0,12 g | Cis-1-(3-Chlorallyl)-3,5,7-triaza-1-azonia-adamantan-chlorid |
| 0,10 g | Parfüm |
| 23,00 g | Glycerin (86prozentig) |
| 72,22 g | Wasser |
| 100,00 g | |

| | Beispiel 16: Festigendes Haarstylinggel |
|---|---|
| 1,53 g | anorganisch-organisches Hybridprepolysiloxan aus Mercaptopropyltriethoxysilan und Salzsäure (System 9.)) |
| 2,50 g | Polyvinylpyrrolidon |
| 2,10 g | Hydroxypropyl-Guar |
| 0,80 g | Hydriertes Rizinusöl, oxethyliert mit 45 Mol Ethylenoxid |
| 0,45 g | Natriumbenzoat |
| 0,30 g | Hydroxyethylcellulose |
| 0,20 g | Parfüm |
| 0,09 g | Natriumformiat |
| 0,05 g | Mica/Titanoxid/Zinnoxid-Pulver (Soloron® Silver Sparkle der Firma Merck/Deutschland) |
| 91,98 g | Wasser |
| 100,00 g | |

| | **Beispiel 17:** Haarfestigendes Liquid-Gel |
|---|---|
| 2,95 g | anorganisch-organisches Hybridprepolysiloxan aus 3-Glycidoxypropyl-trimethoxysilan, Tetramethoxysilan, Tributoxyaluminium, Tetrapropoxyzirkonium und Triethanolamin (System 3.)) |
| 0,35 g | Polyacrylsäure |
| 0,15 g | Hydroxyethylcellulose |
| 0,26 g | 2-Aminopropanol |
| 1,80 g | Polyoxyethylen-(20)-sorbitanmonopalmitat |
| 1,35 g | Polyethylenglykol-(45) |
| 1,05 g | Hydroxyethylcellulose |
| 0,20 g | 1,2-Dibrom-2,4-dicyanobutan |
| 0,30 g | Parfüm |

(fortgesetzt)

| **Beispiel 17:** Haarfestigendes Liquid-Gel | |
|---|---|
| 91,59 g | Wasser |
| 100,00 g | |

Die vorstehend genannten Mittel werden in der Weise angewendet, daß sie, sofern es sich um flüssige Festiger bzw. Lotionen handelt, nach der Haarwäsche auf das handtuchtrockene Haar gegeben und gleichmäßig verteilt werden. Anschließend wird die Frisur erstellt und mit einem Fön bei mittlerer Heizstufe oder mit einem thermostatisierbaren Haartrockner bei 40 bis 50°C für 15 bis 20 Minuten getrocknet.

Gele oder Sprays werden in der Weise angewendet, daß nach einer Haarwäsche zunächst die Frisur erstellt und das Haar getrocknet wird. Anschließend wird das Gel oder Spray auf das trockene Haar appliziert. Danach wird entweder an der Luft oder mit einem Fön bei mittlerer Heizstufe oder mit einem thermostatisierbaren Haartrockner bei 40 bis 50°C für 15 bis 20 Minuten getrocknet.

| **Beispiel 18:** Fönlotion mit UV-Schutz | |
|---|---|
| 1,40 g | anorganisch-organisches Hybridprepolysiloxan aus Mercaptopropyltriethoxysilan, Vinyltriethoxysilan und wässrige Salzsäure (1N) (System 11.)) |
| 1,50 g | Vinylpyrrolidon/Vinylacetat Copolymer |
| 0,20 g | Parfüm |
| 0,15 g | Glycerin (85prozentig) |
| 0,10 g | 2-Hydroxy-4-methoxybenzophenon |
| 42,90 g | Wasser |
| 51,60 g | Ethanol |
| 100,0 g | |

| **Beispiel 19:** Fönlotion mit Antischuppenwirkung | |
|---|---|
| 1,40 g | anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 1,50 g | Vinylpyrrolidon/Vinylacetat Copolymer |
| 0,20 g | Parfüm |
| 1,15 g | 1,2- Propylenglycol |
| 0,40 g | 1-Hydroxy-4-methyl-6-(2,4,4-trimethylpentyl)-2(1H)pyridon Monoethanolaminsalz |
| 42,60 g | Wasser |
| 50,60 g | Ethanol |
| 100,0 g | |

| **Beispiel 20:** Farbfestiger | |
|---|---|
| 0,23 g | Anorg.-org. Hybridprepolymer aus Mercaptopropyltriethoxysilan und Salzsäure (System 9.)) |
| 2,50 g | Vinylacetat/Crotonsäure/Polyglykol Copolymer |
| 0,20 g | Parfüm |
| 0,07 g | 1-Amino-4-(2',3'-dehydroxypropyl)amino-5-chlor-2-nitrobenzol |
| 0,05 g | Basic Brown 17 (C. I. 12 251) |
| 0,01 g | Basic Blue 7 (C. I. 42 595) |
| 0,0023 | Basic Violett 14 (C. I. 42 510) |
| 46,94 g | Wasser |
| 50,00 g | Ethanol |
| 100,0 g | |

| Beispiel 21: Farbfestiger | |
|---|---|
| 1,23 g | anorganisch-organisches Hybridprepolysiloxan aus Mercaptopropyltriethoxysilan, Vinyltriethoxysilan und wässrige Salzsäure (1N) (System 11.)) |
| 1,50 g | Vinylacetat/Crotonsäure Copolymer |
| 0,20 g | Parfüm |
| 0,09 g | 3-(((2-Nitro-4-(trifluormethyl)phenyl)amino)-1,2-propandiol |
| 46,94 g | Wasser |
| 50,08 g | Ethanol |
| 100,0 g | |

| Beispiel 22: Farbfestiger | |
|---|---|
| 1,23 g | Anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 1,50 g | Vinylacetat/Crotonsäure/Polyglykol Copolymer |
| 0,20 g | Parfüm |
| 19,092 g | Colorona Carmine Red |
| 38,04 g | Wasser |
| 40,00 g | Ethanol |
| 100,0 g | |

| Beispiel 23: Farb-Schaumfestiger | |
|---|---|
| 3,00 g | anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 1,00 g | Vinylpyrrolidon/Methylaminoethylmethacrylat Copolymer |
| 0,07 g | 1-Amino-4-(2',3'-dehydroxypropyl)amino-5-chlor-2-nitrobenzol |
| 0,05 g | Basic Brown 17 (C. I. 12 251) |
| 0,01 g | Basic Blue 7 (C. I. 42 595) |
| 18,66 g | Ethanol |
| 70,74 g | Wasser |
| 100,0 g | |

| Beispiel 24: Farb-Schaumfestiger | |
|---|---|
| 3,00 g | anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 1,00 g | Vinylpyrrolidon/Methylaminoethylmethacrylat Copolymer |
| 0,11 g | 3-(((2-Nitro-4-(trifluormethyl)phenyl)amino)-1,2-propandiol |
| 0,20 g | 1,2-Propylenglykol |
| 0,17 g | Parfüm |
| 0,10 g | Cetyltrimethylammoniumchlorid |
| 6,00 g | Propan/Butan (5,0 bar) |
| 18,66 g | Ethanol |
| 70,76 g | Wasser |
| 100,0 g | |

| Beispiel 25: Farb-Schaumfestiger | |
|---|---|
| 3,00 g | anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 1,00 g | Vinylpyrrolidon/Methylaminoethylmethacrylat Copolymer |
| 20,10 g | Colorona Carmine Red |
| 0,20 g | 1,2-Propylenglykol |
| 0,17 g | Parfüm |
| 0,10 g | Cetyltrimethylammoniumchlorid |
| 6,00 g | Propan/Butan (5,0 bar) |
| 18,66 g | Ethanol |
| 50,77 g | Wasser |
| 100,0 g | |

| Beispiel 26: Farb-Haargel | |
|---|---|
| 2,76 g | anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 0,40 g | Polyacrylsäure |
| 0,10 g | Hydroxypropylmethylcellulose |
| 0,80 g | Polyoxyethylen-(20)-sorbitanmonopalmitat |
| 0,50 g | Polyoxyethylen-(25)-p-aminobenzoesäure |
| 0,12 g | Cis-1-(3-Chlorallyl)-3,5,7-triaza-1-azoniaadamantan-chlorid |
| 20,20 g | Colorona Carmine Red |
| 0,10 g | Parfüm |
| 18,00 g | Glycerin (86prozentig) |
| 57,22 g | Wasser |
| 100,0 g | |

| Beispiel 27 : Farbloser Nagellack | |
|---|---|
| 6,0 g | anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 18,0 g | Nitrocellulose (alkoholfeucht 65:35) |
| 4,0 g | Dibuthylphthalat |
| 2,0 g | Campher |
| 40,0 g | Butylacetat |
| 30,0 g | Ethylacetat |
| 100,0 g | |

| Beispiel 28: Farbiger Nagellack | |
|---|---|
| 6,0 g | anorg.-org. Hybridprepolymer aus 3-Glycidoxypropyl-trimethoxysilan, Tetramethoxysilan, Tributoxyaluminium, Tetrapropoxyzirkonium und Triethanolamin (System 3.)) |
| 4,0 g | Dibuthylphthalat |
| 2,0 g | Trikresylphosphat |
| 20,0 g | Ethylenglykolmonomethylether |
| 2,0 g | Diethylenglykolmonomethylether |
| 28,0 g | Methylenchlorid |
| 14,5 g | Ethanol |

(fortgesetzt)

| **Beispiel 28:** Farbiger Nagellack | |
|---|---|
| 9,0 g | Butylacetat |
| 6,0 g | Ethylacetat |
| 2,5 g | Colorona Carmine Red |
| 100,0 g | |

**Patentansprüche**

1. Verwendung von mindestens einem anorganisch-organischen Hybridprepolymer in kosmetischen Mitteln, wobei das Hybridprepolymer gebildet ist durch

   - hydrolytische Vorkondensation, gegebenenfalls in Anwesenheit mindestens eines Kondensationskatalysators, von mindestens einem organofunktionellem Silan der Formel (I):

$$RSiX_3 \qquad\qquad (I)$$

   wobei X für eine hydrolysierbare und kondensierbare Gruppe und R für einen vernetzbaren organischen Rest steht, in Kombination mit netzwerkmodifizierenden Verbindungen ausgewählt aus

   a) Alkyl-, Alkoxy-, Halogen-, Acyloxy-, Hydroxy-, Oxyhalogen- oder Hydroxyhalogenverbindungen von Metallen der Übergangselemente, insbesondere der IV. Nebengruppe wie Titan oder Zirkonium oder der III. Hauptgruppe wie Aluminium und/oder
   b) Verbindungen der Formel $SiX_4$ und/oder
   c) Verbindungen der Formel $SiR'X_2R$, wobei R' für eine Alkyl- oder Arylgruppe steht und R und X die oben angegebenen Bedeutungen haben oder durch

   - hydrolytische Vorkondensation, gegebenenfalls in Anwesenheit mindestens eines Kondensationskatalysators, von mindestens einem organofunktionellem Silan der Formel (I), wobei X für eine hydrolysierbare und kondensierbare Gruppe und R für einen vernetzbaren organischen Rest steht, wobei der vernetzbare organische Rest R mindestens einen vernetzbaren Substituenten enthält, der ausgewählt ist aus Alkinyl, Arylalkinyl, Alkinylaryl, Halogenen, Aldehyd-, Mercapto-, Cyano-, Alkoxy- oder Methacryloxygruppen.

2. Verwendung von mindestens einem anorganisch-organischen Hybridprepolymer gemäß Anspruch 1 oder mindestens einem vernetzten anorganisch-organischen Hybridpolymer zur Haarbehandlung.

3. Verwendung nach einem der vorgenannten Ansprüche, **dadurch gekennzeichnet, daß** der hydrolysierbare Rest X ausgewählt ist aus Alkoxy-, Aryloxy-, Acyloxy-, Alkylcarbonyl-, Alkoxycarbonylgruppen, Halogen, Wasserstoff oder substituierten oder unsubstituierten Aminogruppen.

4. Verwendung nach einem der vorgenannten Ansprüche, **dadurch gekennzeichnet, daß** das Silan der Formel (I) ausgewählt ist aus Vinyltrialkoxysilan, Vinyltriacetoxysilan, Aminopropyltrialkoxysilan, Isocyanatopropyltrialkoxysilan, Mercaptopropyltrialkoxysilan, Vinyltrichlorsilan, Allyltrialkoxysilan, Allyltriacetoxysilan, 3-Isocyanatooxypropyltrialkoxysilan, Methacryloxypropenyltrialkoxysilan, 3-Methacrylcarbonyloxypropyltrialkoxysilan, p-Aminophenyltrialkoxysilan, 3-Aminopropyltrialkoxysilan, 3-Cyanopropyltrialkoxysilan, 4-Mercaptobutyltrialkoxysilan, 6-Mercaptohexyltrialkoxysilan, 3-Mercaptopropyltrialkoxysilan, 3-(Ethylendiamino)propyltrialkoxysilan, 3-(Diethylentriamino)propyltrialkoxysilan, 3-Glycidoxypropyltrialkoxysilan, 2-[4-(1,2-Epoxycyclohexyl)]ethyltrialkoxysilan und 3-(Trialkoxysilyl)propylbernsteinsäureanhydrid, wobei Alkoxygruppen Methoxy- oder Ethoxygruppen bedeuten.

5. Verwendung von anorganisch-organischen Hybridprepolymeren oder vernetzten anorganisch-organischen Hybridpolymeren als Träger für kosmetische oder pharmazeutische Wirkstoffe.

6. Kosmetisches Mittel mit einem Gehalt an mindestens einem anorganisch-organischen Hybridprepolymer gemäß Anspruch 1 in einer geeigneten kosmetischen Grundlage.

7. Mittel nach Anspruch 6, **dadurch gekennzeichnet, daß** es 0,01 bis 40 Gew.% des Hybridprepolymers enthält.

8. Mittel nach einem der Ansprüche 6 bis 7, **dadurch gekennzeichnet, daß** es zusätzlich mindestens ein filmbildendes, haarfestigendes Polymer enthält.

9. Verfahren zur Haarbehandlung, bei welchem

   a) ein noch nicht vernetztes Vorkondensationsprodukt eines anorganisch-organischen Hybridpolymers in einer geeigneten kosmetischen Grundlage auf das Haar aufgebracht wird und
   b) anschließend zum Hybridpolymer vernetzt wird.

**Claims**

1. Use of at least one inorganic-organic hybrid prepolymer in cosmetic compositions, where the hybrid prepolymer is formed by

   - hydrolytic precondensation, optionally in the presence of at least one condensation catalyst, of at least one organofunctional silane of the formula (I): $RSiX_3$ (I)
   where X is a hydrolysable and condensable group and R is a crosslinkable organic radical, in combination with network-modifying compounds chosen from

      a) alkyl, alkoxy, halogen, acyloxy, hydroxy, oxyhalogen or hydroxyhalogen compounds of metals of the transition elements, in particular of subgroup IV, such as titanium or zirconium, or of main group III, such as aluminium and/or
      b) compounds of the formula $SiX_4$ and/or
      c) compounds of the formula $SiR'X_2R$, where R' is an alkyl or aryl group, and R and X have the meanings given above, or by

   - hydrolytic precondensation, optionally in the presence of at least one condensation catalyst, of at least one organofunctional silane of the formula (I), where X is a hydrolysable and condensable group and R is a crosslinkable organic radical, where the crosslinkable organic radical R contains at least one crosslinkable substituent which is chosen from alkynyl, arylalkynyl, alkynylaryl, halogens, aldehyde, mercapto, cyano, alkoxy or methacryloxy groups.

2. Use of at least one inorganic-organic hybrid prepolymer according to Claim 1 or at least one crosslinked inorganic-organic hybrid polymer for the treatment of hair.

3. Use according to either of the preceding claims, **characterized in that** the hydrolysable radical X is chosen from alkoxy, aryloxy, acyloxy, alkylcarbonyl, alkoxycarbonyl groups, halogen, hydrogen or substituted or unsubstituted amino groups.

4. Use according to one of the preceding claims, **characterized in that** the silane of the formula (I) is chosen from vinyltrialkoxysilane, vinyltriacetoxysilane, aminopropyltrialkoxysilane, isocyanatopropyltrialkoxysilane, mercaptopropyltrialkoxysilane, vinyltrichlorosilane, allyltrialkoxysilane, allyltriacetoxysilane, 3-isocyanatooxypropyltrialkoxysilane, methacryloxypropenyltrialkoxysilane, 3-methacrylcarbonyloxypropyltrialkoxysilane, p-aminophenyltrialkoxysilane, 3-aminopropyltrialkoxysilane, 3-cyanopropyltrialkoxysilane, 4-mercaptobutyltrialkoxysilane, 6-mercaptohexyltrialkoxysilane, 3-mercaptopropyltrialkoxysilane, 3-(ethylenediamino)propyltrialkoxysilane, 3-(diethylenetriamino)propyltrialkoxysilane, 3-glycidoxypropyltrialkoxysilane, 2-[4-(1,2-epoxycyclohexyl)]ethyltrialkoxysilane and 3-(trialkoxysilyl)propyl succinic anhydride, where alkoxy groups are methoxy or ethoxy groups.

5. Use of inorganic-organic hybrid prepolymers or crosslinked inorganic-organic hybrid polymers as carriers for cosmetic or pharmaceutical active ingredients.

6. Cosmetic composition with a content of at least one inorganic-organic hybrid prepolymer according to Claim 1 in a suitable cosmetic base.

7. Composition according to Claim 6, **characterized in that** it comprises 0.01 to 40% by weight of the hybrid prepolymer.

8. Composition according to either Claim 6 or 7, **characterized in that** it additionally comprises at least one film-forming, hair-setting polymer.

9. Method for the treatment of hair, in which

   a) an as yet uncrosslinked precondensation product of an inorganic-organic hybrid polymer in a suitable cosmetic base is applied to the hair and
   b) is then crosslinked to give the hybrid polymer.


**Revendications**

1. Utilisation d'au moins un prépolymère hybride inorganique-organique dans des compositions cosmétiques, le prépolymère hybride étant formé par

   - précondensation hydrolytique, éventuellement en présence d'au moins un catalyseur de condensation, d'au moins un silane organofonctionnel de formule (I) : $RSiX_3$ (I)
   dans laquelle X représente un groupe hydrolysable et condensable et R représente un radical organique réticulable, en combinaison avec des composés modificateurs de réseau choisis parmi

      a) des composés alkyle, alcoxy, halogénés, acyloxy, hydroxy, oxyhalogénés ou hydroxyhalogénés de métaux des éléments de transition, en particulier du groupe secondaire IV, tels que le titane ou le zirconium, ou du groupe principal III, tels que l'aluminium, et/ou
      b) des composés de formule $SiX_4$ et/ou
      c) des composés de formule $SiR'X_2R$, dans laquelle R' représente un groupe alkyle ou aryle .et R et X présentent les significations indiquées ci-dessus, ou par

   - précondensation hydrolytique, éventuellement en présence d'au moins un catalyseur de condensation, d'au moins un silane organofonctionnel de formule (I), dans laquelle X représente un groupe hydrolysable et condensable et R représente un radical organique réticulable, le radical organique réticulable R renfermant au moins un substituant réticulable, qui est choisi parmi des groupes alcynyle, arylalcynyle, alcynylaryle, halogène, aldéhyde, mercapto, cyano, alcoxy ou méthacryloxy.

2. Utilisation d'au moins un prépolymère hybride inorganique-organique selon la revendication 1 ou d'au moins un polymère hybride inorganique-organique réticulé pour le traitement des cheveux.

3. Utilisation selon l'une quelconque des revendications précédentes, **caractérisée en ce que** le radical hydrolysable X est choisi parmi des groupes alcoxy, aryloxy, acyloxy, alkylcarbonyle, alcoxycarbonyle, un halogène, un hydrogène ou des groupes amino substitués ou non substitués.

4. Utilisation selon l'une quelconque des revendications précédentes, **caractérisée en ce que** le silane de formule (I) est choisi parmi un vinyltrialcoxysilane, le vinyltriacétoxysilane, un aminopropyltrialcoxysilane, un isocyanatopropyltrialcoxysilane, un mercaptopropyltrialcoxysilane, le vinyltrichlorosilane, un allyltrialcoxysilane, l'allyltriacétoxysilane, un 3-isocyanatooxypropyltrialcoxysilane, un méthacryloxypropényltrialcoxysilane, un 3-méthacrylcarbonyloxypropyltrialcoxysilane, un p-aminophényltrialcoxysilane, un 3-aminopropyltrialcoxysilane, un 3-cyanopropyltrialcoxysilane, un 4-mercaptobutyltrialcoxysilane, un 6-mercaptohexyltrialcoxysilane, un 3-mercaptopropyltrialcoxysilane, un 3-(éthylènediamino)propyltrialcoxysilane, un 3-(diéthylènetriamino)-propyltrialcoxysilane, un 3-glycidoxypropyltrialcoxysilane, un 2-[4-(1,2-époxycyclohexyl)]-éthyltrialcoxysilane et un anhydride 3-(trialcoxysilyl)propylsuccinique, les groupes alcoxy représentant des groupes méthoxy ou éthoxy.

5. Utilisation de prépolymères hybrides inorganiques-organiques ou de polymères hybrides inorganiques-organiques réticulés en tant que supports pour des ingrédients actifs cosmétiques ou pharmaceutiques.

6. Composition cosmétique contenant au moins un prépolymère hybride inorganique-organique selon la revendication 1 dans une base cosmétique appropriée.

**7.** Composition selon la revendication 6, **caractérisée en ce qu'**elle comprend de 0,01 à 40 % en poids du prépolymère hybride.

**8.** Composition selon l'une quelconque des revendications 6 à 7, **caractérisée en ce qu'**elle comprend en outre au moins un polymère filmogène fixateur des cheveux.

**9.** Procédé pour le traitement des cheveux, dans lequel

a) un produit de précondensation, pas encore réticulé, d'un polymère hybride inorganique-organique dans une base cosmétique appropriée est appliqué sur les cheveux, et
b) est ensuite réticulé pour donner lieu au polymère hybride.